(19) Europäisches Patentamt
European Patent Office
Office européen des brevets

(11) Veröffentlichungsnummer : **0 316 734 B1**

(12) **EUROPÄISCHE PATENTSCHRIFT**

(45) Veröffentlichungstag der Patentschrift : **03.07.91 Patentblatt 91/27**

(51) Int. Cl.$^5$ : **C07D 271/08**, C07D 413/04, A01N 47/36, C07C 265/12, C07C 331/26

(21) Anmeldenummer : **88118633.2**

(22) Anmeldetag : **09.11.88**

(54) **Furazanylharnstoffe.**

Die Akte enthält technische Angaben, die nach dem Eingang der Anmeldung eingereicht wurden und die nicht in dieser Patentschrift enthalten sind.

(30) Priorität : **17.11.87 DE 3738946**

(43) Veröffentlichungstag der Anmeldung : **24.05.89 Patentblatt 89/21**

(45) Bekanntmachung des Hinweises auf die Patenterteilung : **03.07.91 Patentblatt 91/27**

(84) Benannte Vertragsstaaten : **BE CH DE ES FR GB IT LI NL**

(56) Entgegenhaltungen :
**EP-A- 229 630**
**EP-A- 0 132 680**
**US-A- 3 937 726**

(73) Patentinhaber : **BAYER AG**
**W-5090 Leverkusen 1 Bayerwerk (DE)**

(72) Erfinder : **Sirrenberg, Wilhelm, Dr.**
**Wuppertaler Strasse 21**
**W-4322 Sprockhoevel (DE)**
Erfinder : **Marhold, Albrecht, Dr.**
**Carl-Duisberg-Strasse 329**
**W-5090 Leverkusen 1 (DE)**
Erfinder : **Wachendorff-Neumann, Ulrike, Dr.**
**Hermann-Loens-Strasse 16**
**W-5090 Leverkusen 3 (DE)**

EP 0 316 734 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue 1-Arylfurazanyl-3-(2-alkyl-4-halogenalkoxy-phenyl)-(thio)harnstoffe, Verfahren und neue Zwischenprodukte zu ihrer Herstellung sowie ihre Verwendung als Schädlingsbekämpfungsmittel, insbesondere als Akarizide.

Es ist bereits bekannt, daß bestimmte substituierte Furazane, wie z.B. 1-(4-Phenyl-1,2,5-oxadiazol-3-yl)-3-(4-trifluormethoxy-phenyl)-harnstoff und 1-(4-Phenyl-1,2,5-oxadiazol-3-yl)-3-(4-(2-chlor-1,1,2-trifluor-ethoxy)-phenyl)-harnstoff, akarizide Eigenschaften aufweisen (vgl. EP-A 132 680). In der US-Patentschrift Nr. 3 937 762 wurde die Verbindung 2-Methyl-4-(1,1-difluor-2,2-dichlorethyl)-phenylisocyanat als Vorprodukt für herbizid wirksame Harnstoffderivate beschrieben.

Es wurden nun die neuen 1-Arylfurazanyl-3-(2-alkyl-4-halogenalkoxy-phenyl)-(thio)harnstoffe der allgemeinen Formel (I)

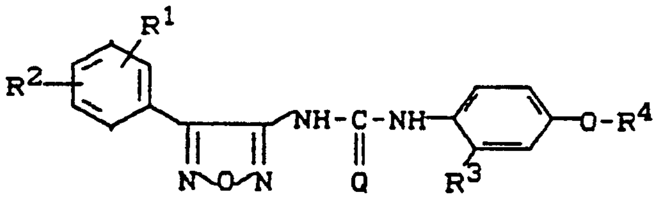

(I)

in welcher

| | |
|---|---|
| Q | für Sauerstoff oder Schwefel steht, |
| $R^1$ | für Wasserstoff, Halogen, Alkyl, Halogenalkyl, Alkoxy, Halogenalkoxy, Alkylthio oder Halogenalkylthio steht und |
| $R^2$ | für Wasserstoff, Halogen, Alkyl oder Alkoxy steht, oder |
| $R^1$ und $R^2$ | zusammen für gegebenenfalls durch Halogen substituiertes alkylendioxy stehen, |
| $R^3$ | für Alkyl steht und |
| $R^4$ | für durch Fluor und gegebenenfalls zusätzlich durch Chlor und/oder Brom substituiertes Alkyl steht, gefunden. |

Weiter wurde gefunden, daß man die neuen 1-Arylfurazanyl-3-(2-alkyl-4-halogenalkoxy-phenyl)-(thio)harnstoffe der allgemeinen Formel (I) erhält, wenn man

(a) 3-Amino-4-aryl-1,2,5-oxadiazole ("Arylfurazanylamine") der allgemeinen Formel (II),

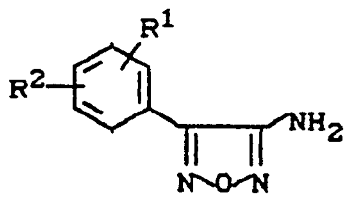

(II)

in welcher
$R^1$ und $R^2$ die oben angegebenen Bedeutungen haben,
mit 2-Alkyl-4-halogenalkoxy-phenyl-iso(thio)cyanaten der allgemeinen Formel (III)

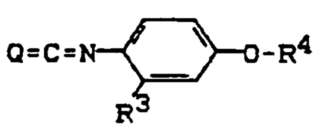

(III)

in welcher
Q, $R^3$ und $R^4$ die oben angegebenen Bedeutungen haben,
gegebenenfalls in Gegenwart von Katalysatoren und gegebenenfalls in Gegenwart von Verdünnungsmitteln umsetzt, oder

(b) 3-Iso(thio)cyanato-4-aryl-1,2,5-oxadiazole (Arylfurazanyliso(thio)cyanate") der allgemeinen Formel (IV)

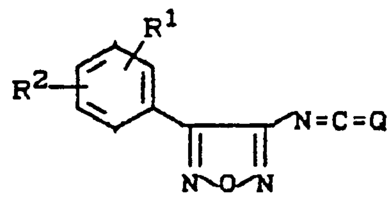

(IV)

in welcher
Q, $R^1$ und $R^2$ die oben angegebenen Bedeutungen haben,
mit 2-Alkyl-4-halogenalkoxy-anilinen der allgemeinen Formel (V)

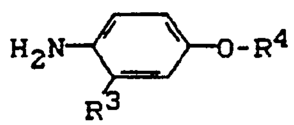

(V)

in welcher
$R^3$ und $R^4$ die oben angegebenen Bedeutungen haben,
gegebenenfalls in Gegenwart von Katalysatoren und gegebenenfalls in Gegenwart von Verdünnungsmitteln umsetzt.

Alkyl $R^1$, $R^2$, $R^3$ and $R^4$ ist geradkettig oder verzweigt und enthält vorzugsweise 1 bis 6, insbesondere 1 bis 4, Kohlenstoffatome. Beispielhaft seien Methyl, Ethyl, n-Propyl, Isopropyl, n-Butyl, Isobutyl, sec-Butyl und tert-Butyl genannt. Methyl und Ethyl sind besonders bevorzugt.

Halogenalkyl $R^1$ ist geradkettig oder verzweigt und enthält vorzugsweise 1 bis 4, insbesondere 1 oder 2, Kohlenstoffatome und vorzugsweise 1 bis 3 Halogenatome, wobei die Halogenatome gleich oder verschieden sind und als Halogenatome vorzugsweise Fluor, Chlor oder Brom, insbesondere Fluor, stehen. Beispielhaft seien Difluormethyl, Trifluormethyl, Chlordifluormethyl und Fluordichlormethyl genannt. Trifluormethyl wird besonders bevorzugt.

Alkoxy und Alkylthio $R^1$ und Alkoxy $R^2$ enthalten im Alkylteil geradkettiges oder verzweigtes Alkyl mit vorzugsweise 1 bis 6, insbesondere 1 bis 4, Kohlenstoffatomen.
Beispielhaft seien Methoxy, Ethoxy, n-Propoxy, Isopropoxy, n-Butoxy, Isobutoxy, sec-Butoxy, Methylthio, Ethylthio, n-Propylthio, Isopropylthio, n-Butylthio, Isobutylthio und sec-Butylthio genannt. Methoxy wird besonders bevorzugt.

Halogenalkoxy und Halogenalkylthio $R^1$ enthalten im Alkylteil geradkettiges oder verzweigtes Alkyl mit vorzugsweise 1 bis 4, insbesondere 1 bis 3, Kohlenstoffatomen und vorzugsweise 1 bis 5, insbesondere 2 bis 4, Halogenatome, wobei die Halogenatome gleich oder verschieden sind und als Halogenatome vorzugsweise Fluor, Chlor oder Brom, insbesondere Fluor, stehen. Beispielhaft seien Difluormethoxy, Trifluormethoxy, Tetrafluorethoxy, Chlortrifluorethoxy, Difluormethylthio, Trifluormethylthio, Chlordifluormethylthio, Fluordichlormethylthio, Tetrafluorethylthio, Fluordichlormethylthio, Tetrafluorethylthio and Chlortrifluorethylthio genannt. Trifluormethoxy und Trifluormethylthio werden besonders bevorzugt.

Alkylendioxy in der Definition von $R^1$ und $R^2$ enthält vorzugsweise 1 bis 3, insbesondere 1 oder 2, Kohlenstoffatome und kann geradkettiges oder verzweigtes Alkylen enthalten, welches gegebenenfalls durch Halogen (vorzugsweise Fluor und/oder Chlor) substituiert ist. Beispielhaft seien Methylendioxy, Difluormethylendioxy, Dimethylendioxy, Trifluordimethylendioxy, Tetrafluordimethylendioxy und Chlortrifluordimethylendioxy genannt.

Halogen steht, wo nicht anders erläutert, für Fluor, Chlor, Brom oder Iod, vorzugsweise für Fluor oder Chlor.

Q steht bevorzugt für Sauerstoff.

Die neuen Verbindungen der allgemeinen Formel (I) verfügen über Eigenschaften, die ihre Verwendung als Schädlingsbekämpfungsmittel ermöglichen ; sie zeigen gute insektizide Wirkung und zeichnen sich insbesondere durch sehr starke akarizide Wirksamkeit aus.

Bevorzugt werden die neuen 1-Arylfurazanyl-3-(2-alkyl-4-halogenalkoxy-phenyl)-(thio)harnstoffe der Formel (I), in welcher

Q für Sauerstoff oder Schwefel steht,

$R^1$ für Wasserstoff, Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_6$-Alkylthio oder für $C_1$-$C_4$-Halogenalkylthio steht und

$R^2$ für Wasserstoff, Halogen, $C_1$-$C_4$-Alkyl oder $C_1$-$C_4$-Alkoxy steht, oder

$R^1$ und $R^2$ zusammen für gegebenenfalls durch Fluor und/oder Chlor substituiertes $C_1$-$C_3$-Alkylendioxy stehen,

$R^3$ für $C_1$-$C_4$-Alkyl steht und

$R^4$ für durch Fluor und gegebenenfalls zusätzlich durch Chlor und/oder Brom substituiertes $C_1$-$C_6$-Alkyl steht.

Besonders bevorzugt werden die neuen 1-Arylfurazanyl-3-(2-alkyl-4-halogenalkoxy-phenyl)-(thio)harnstoffe der Formel (I), in welcher

Q für Sauerstoff oder Schwefel (vorzugsweise Sauerstoff) steht,

$R^1$ für Wasserstoff, Fluor, Chlor, Brom, $C_1$-$C_4$-Alkyl, für durch Fluor und/oder Chlor substituiertes Methyl, für $C_1$-$C_4$-Alkoxy, für durch Fluor und/oder Chlor substituiertes $C_1$-$C_3$-Alkoxy, für $C_1$-$C_4$-Alkylthio oder für durch Fluor und/oder Chlor substituiertes $C_1$-$C_4$-Alkylthio steht und

$R^2$ für Wasserstoff, Fluor, Chlor, Brom, Methyl oder Methoxy steht, oder

$R^1$ und $R^2$ zusammen für gegebenenfalls durch Fluor und/oder Chlor substituiertes $C_1$-$C_2$-Alkylendioxy stehen,

$R^3$ für Methyl steht und

$R^4$ für durch Fluor und gegebenenfalls zusätzlich durch Chlor substituiertes $C_1$-$C_4$-Alkyl steht.

Ganz besonders bevorzugt werden die Verbindungen der Formel (I), in welcher

$R^1$ für Wasserstoff, Fluor, Chlor, Methyl, Trifluormethyl oder Methoxy steht,

$R^2$ für Wasserstoff, Fluor oder Chlor steht,

$R^3$ für Methyl steht,

$R^4$ für $-CF_3$ oder $-CF_2CHFCl$ steht und

Q für Sauerstoff oder Schwefel (vorzugsweise Sauerstoff) steht.

Beispiele für Verbindungen der Formel (I) sind in der nachstehenden Tabelle 1 aufgeführt – vgl. auch die Herstellungsbeispiele.

Tabelle 1: Beispiele für die Verbindungen der Formel (I)

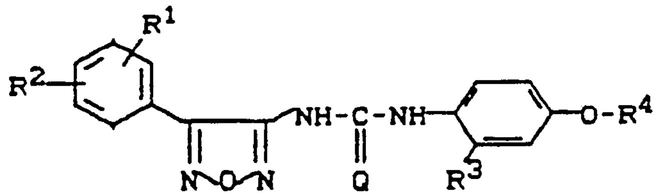

(I)

| Q | $R^1$ | $R^2$ | $R^3$ | $R^4$ |
|---|---|---|---|---|
| O | H | H | $CH_3$ | $CHF_2$ |
| S | H | H | $CH_3$ | $CHF_2$ |
| O | 2-Br | H | $CH_3$ | $CF_3$ |
| O | 2-Cl | 4-Cl | $CH_3$ | $CF_3$ |
| O | 2-F | 6-F | $CH_3$ | $CF_3$ |
| O | 2-$CH_3$ | H | $CH_3$ | $CF_3$ |
| O | 4-$CF_3$ | H | $CH_3$ | $CF_3$ |
| O | 4-$OCH_3$ | H | $CH_3$ | $CHF_2$ |
| O | 3-$OCH_3$ | 4-$OCH_3$ | $CH_3$ | $CF_3$ |
| O | 4-$SCH_3$ | H | $CH_3$ | $CF_3$ |
| O | 4-$OCF_3$ | H | $CH_3$ | $CF_3$ |
| O | 4-$SCF_3$ | H | $CH_3$ | $CF_3$ |
| O | H | H | $CH_3$ | $CF_2CHF_2$ |
| O | H | H | $CH_3$ | $CH_2CF_3$ |
| O | H | H | $CH_3$ | $CF_2CF_3$ |
| O | H | H | $C_2H_5$ | $CF_2CHFCl$ |
| O | H | H | $CH_3$ | $CF_2CHCl_2$ |
| O | H | H | $CH_3$ | $CF_2CCl_3$ |

Tabelle 1 - Fortsetzung

| Q | $R^1$ | $R^2$ | $R^3$ | $R^4$ |
|---|---|---|---|---|
| O | H | H | $CH_3$ | $CF_2CHBr_2$ |
| O | H | H | $CH_3$ | $CF_2CHFBr$ |
| O | H | H | $CH_3$ | $CF_2CHFCF_3$ |
| S | H | H | $CH_3$ | $CF_2CHFCF_3$ |
| S | H | H | $CH_3$ | $CH_2CF_3$ |
| O | H | H | $CH_3$ | $CF_2Cl$ |
| S | H | H | $CH_3$ | $CF_2Cl$ |

Verwendet man nach Verfahrensvariante (a) 3-Amino-4-phenyl-1,2,5-oxadiazol und 2-Methyl-4-difluormethoxyphenylisocyanat als Ausgangsstoffe, so kann der Reaktionsablauf durch das folgende Formelschema wiedergegeben werden :

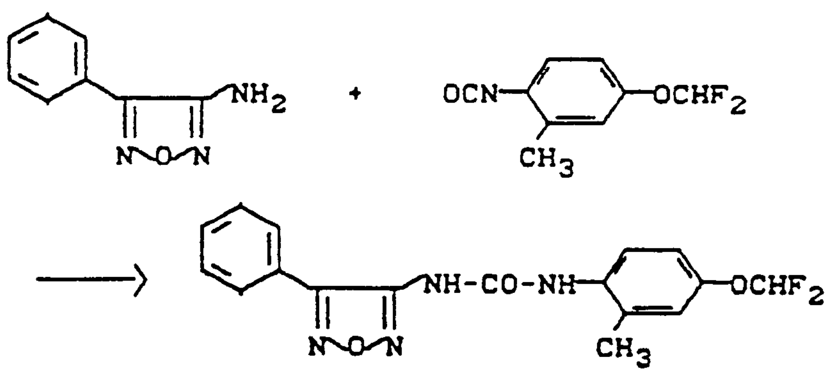

Verwendet man nach Verfahrensvariante (b) 3-Isocyanato-4-phenyl-1,2,5-oxadiazol und 2-Methyl-4-trifluormethoxy-anilin als Ausgangsstoffe, so kann der Reaktionsablauf durch das folgende Formelschema wiedergegeben werden :

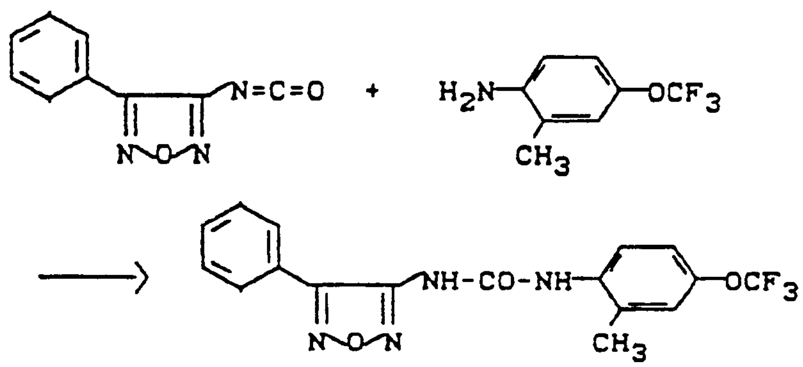

Die als Ausgangsstoffe zu verwendenden 3-Amino-4-aryl-1,2,5-oxadiazole der Formel (II) sind bekannt und/oder können nach an sich bekannten Verfahren hergestellt werden (vgl. J. Prakt. Chem. 315 (1973), 791-795 ; US-P 3 594 388).

Die Verbindungen der Formel (II) können nach üblichen Methoden in Verbindungen der Formel (IV) umgewandelt werden, z.B. durch Umsetzung mit Phosgen bzw. Thiophosgen in Verdünnungsmitteln, wie z.B. Toluol, o-Dichlorbenzol und/oder Pyridin, bei Temperaturen zwischen 20°C und 200°C (vgl. EP-A 132 680, DE-OS 34 09 887).

Die weiter als Ausgangsstoffe zu verwendenen 2-Alkyl-4-halogenalkoxy-aniline der Formel (V) sind ebenfalls bekannt und/oder können nach an sich bekannten Verfahren hergestellt werden (vgl. DE-OS 21 13 978, DE-OS 31 35 926, EP-A 136 974, US-P 39 37 726, US-P 40 13 452).

Die Verbindungen der Formel (V) können nach üblichen Methoden in Verbindungen der Formel (III) umgewandelt werden, z.B. durch Umsetzung mit Phosgen bzw. Thiophosgen in Verdünnungsmitteln, wie z.B. Toluol, o-Dichlorbenzol und/oder Pyridin, bei Temperaturen zwischen 20°C und 200°C (vgl. Angew. Chem. 89 (1977), 979-804).

Die Verbindungen der Formel (III), die auf diese Weise erhalten werden, sind noch nicht aus der Literatur bekannt.

Als Verdünnungsmittel kommen beim erfindungsgemäßen Verfahren (Varianten (a) und (b)) praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Tetramethylensulfon.

Als Katalysatoren können für die Umsetzung gemäß Verfahrensvarianten (a) und (b) vorzugsweise tertiäre Amine, wie Triethylamin und 1,4-Diazabicyclo-[2,2,2]-octan, sowie organische Zinn-Verbindungen, wie z.B. Dibutylzinndilaurat verwendet werden. Im allgemeinen bringt der Zusatz des Katalysators keine Vorteile.

Die Reaktionstemperatur kann innerhalb eines größeren Bereiches variiert werden. Im allgemeinen arbeitet man bei der Verfahrensvariante (a) zwischen 20°C und 180°C, vorzugsweise zwischen 40°C und 120°C und bei der Verfahrensvariante (b) zwischen 20°C und 200°C, vorzugsweise zwischen 60°C und 190°C. Die erfindungsgemäßen Verfahrensvarianten werden im allgemeinen bei Normaldruck durchgeführt.

Zur Durchführung der erfindungsgemäßen Verfahrensvarianten werden werden die Ausgangsstoffe gewöhnlich in etwa äquimolaren Mengen eingesetzt. Ein Überschuß der einen oder anderen Reaktionskomponente bringt keine wesentlichen Vorteile. Vorzugsweise setzt man auf 1 Mol der Verbindungen der Formel (II) bzw. (IV) 0,6 bis 1,5 Mol, insbesondere 0,8 bis 1,2 Mol der Verbindung der Formel (III) bzw. (V) ein.

Die Aufarbeitung der Reaktionsprodukte geschieht nach üblichen Methoden, z.B. durch Absaugen des ausgefallenen Produktes oder durch Herauslösung von unerwünschten Nebenprodukten aus dem Reaktionsgemisch. Zur Charakterisierung dient der Schmelzpunkt.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum,

Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Verbindungen der Formel (I) zeichnen sich durch eine starke insektizide und besonders durch eine starke akarizide Wirksamkeit aus, insbesondere gegen Eier (ovizide Wirkung) und gegen die Larven (larvizide Wirkung) der Schädlinge.

Sie zeigen insbesondere beim Einsatz als Akarizide eine hervorragende Wirkung auf Eier und Larven von gemeiner Spinnmilbe (Tetranychus urticae) und Obstbaumspinnmilbe (Panonychus ulmi) und sind praktisch gegen alle Entwicklungsstadien von Spinnmilben sehr gut wirksam.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cylcohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche beinormaler Temperatur und unter Normaldruck gasförmig sind, z.B. Aerosol-Treibgase, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit,

Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0.5 und 90%.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Alle Prozentangaben beziehen sich im vorliegenden Text auf Gewichtsprozente, wenn keine andere Angabe gemacht wird.

Herstellungsbeispiele

Beispiel 1

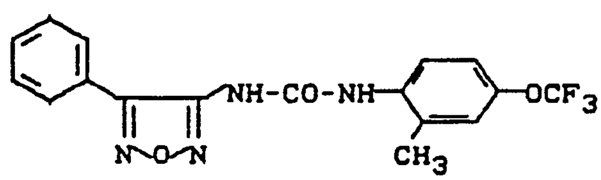

(Verfahrensvariante (b))

Zu einer Lösung von 1,91 g (0,01 Mol) 2-Methyl-4-trifluormethoxy-anilin in 40 ml trockenem Toluol fügt man unter Feuchtigkeitsausschluß bei 60°C 1,87 g (0,01 Mol) 3-Isocyanato-4-phenyl-1,2,5-oxadiazol gelöst in 10 ml trockenem Toluol. Der Ansatz wird eine halbe Stunde bei 80°C gerührt und anschließend im Vakuum eingeengt. Das ausgefallene Produkt wird angesaugt und mit Petrolether gewaschen.

Man erhält 3,5 g (92,5% der Theorie) 1-[(2-Methyl-4-trifluromethoxy)-phenyl]-3-(4-phenyl-1,2,5-oxadiazol-3-yl)-harnstoff vom Schmelzpunkt 198°C.

(Verfahrensvariante (a))

Zu einer Lösung von 6,44 g (0,44 Mol) 3-Amino-4-phenyl-1,2,5-oxadiazol in 20 ml trockenem Dimethylformamid gibt man 9,03 g (0,042 Mol) 2-Methyl-4-trifluormethoxy-phenylisocyanat. Der Ansatz wird zwei Stunden

bei 100°C gerührt ; darauf wird das Lösungsmittel unter Vakuum abgedampft, der Rückstand in siedendem Toluol gelöst, nach Zugabe von etwas Petrolether abgekühlt und das kristallin angefallene Produkt durch Absaugen isoliert.

Man erhält 11,3 g 1-(4-Phenyl-1,2,5,-oxadiazol-3-yl)-3-(2-methyl-4-trifluormethoxy-phenyl)-harnstoff mit einem Gehalt von 74% nach HPLC.

Analog Beispiel 1 und gemäß der allgemeinen Beschreibung der Verfahrensvarianten (a) und (b) können die in der nachstehenden Tabelle 2 aufgeführten Verbindungen der Formel (I) hergestellt werden.

Tabelle 2: Beipiele für die Verbindungen der Formel (I)

| Beisp.-Nr. | Q | $R^1$ | $R^2$ | $R^3$ | $R^4$ | Schmelz-punkt (°C) |
|---|---|---|---|---|---|---|
| 2 | O | 4-F | H | $CH_3$ | $CF_3$ | 190 |
| 3 | O | 4-Cl | H | $CH_3$ | $CF_3$ | 216 |
| 4 | O | 2-Cl | H | $CH_3$ | $CF_3$ | 207 |
| 5 | O | 3-$CF_3$ | H | $CH_3$ | $CF_3$ | 207 |
| 6 | O | 2-F | 4-F | $CH_3$ | $CF_3$ | 204 |
| 7 | O | 4-$CH_3$ | H | $CH_3$ | $CF_3$ | 196 |
| 8 | O | 4-$OCH_3$ | H | $CH_3$ | $CF_3$ | 196 |
| 9 | O | 3-Cl | 4-Cl | $CH_3$ | $CF_3$ | 200 |
| 10 | O | 3-O-$CH_2$-O-4 | | $CH_3$ | $CF_3$ | 196 |
| 11 | O | 2-F | H | $CH_3$ | $CF_3$ | 216 |
| 12 | S | H | H | $CH_3$ | $CF_3$ | 126 |
| 13 | O | H | H | $CH_3$ | $CF_2CHFCl$ | 200 |
| 14 | O | 4-Cl | H | $CH_3$ | $CF_2CHFCl$ | 203 |
| 15 | O | 2-Cl | H | $CH_3$ | $CF_2CHFCl$ | 204 |

Tabelle 2 - Fortsetzung

| Beisp.-Nr. | Q | $R^1$ | $R^2$ | $R^3$ | $R^4$ | Schmelzpunkt (°C) |
|---|---|---|---|---|---|---|
| 16 | O | 2-F | H | $CH_3$ | $CF_2CHFCl$ | 202 |
| 17 | O | 4-F | H | $CH_3$ | $CF_2CHFCl$ | 181 |
| 18 | S | 2-Cl | H | $CH_3$ | $CF_2CHFCl$ | 128 |
| 19 | O | 4-Br | H | $CH_3$ | $CF_2CHFCl$ | 205 |
| 20 | O | 3-$CF_3$ | H | $CH_3$ | $CF_2CHFCl$ | 217 |
| 21 | O | 4-$CH_3$ | H | $CH_3$ | $CF_2CHFCl$ | 168 |
| 22 | O | 4-$OCH_3$ | H | $CH_3$ | $CF_2CHFCl$ | 169 |
| 23 | O | 3-Cl | 4-Cl | $CH_3$ | $CF_2CHFCl$ | 203 |
| 24 | O | 2-F | 4-F | $CH_3$ | $CF_2CHFCl$ | 198 |
| 25 | O | 3-O-$CH_2$-O-4 | | $CH_3$ | $CF_2CHFCl$ | 188 |

Verbindungen der Formel (III)

Herstellungsbeispiel

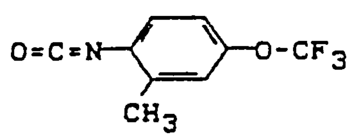

In 500 ml trockenes Toluol gast man bei 0-5°C 50 g Phosgen ein. Anschließend tropft man bei der gleichen Temperatur 95,5 g 2-Methyl-4-trifluormethoxy-anilin in 200 ml trockenem Toluol hinzu. Unter gleichzeitigem Einleiten von Phosgen erhitzt man den Ansatz 3 Stunden auf Rückflußtemperatur. Anschließend wird das überschüssige Phosgen mit Stickstoff ausgeblasen. Das Lösungsmittel wird abdestilliert und der flüssige Rückstand destilliert.

Man erhält 93 g (85,5% der Theorie) 2-Methyl-4-trifluormethoxy-phenylisocyanant vom Siedepunkt $Kp_2$ 54°C und einem Refraktionsindex von $n_D^{20}$ = 1,4626.

Auf die gleiche Weise stellt man die anderen Isocanate dar, z.B.

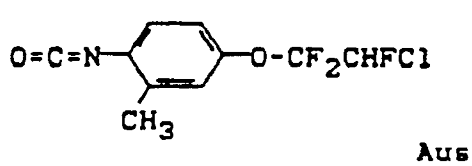

$Kp_2$ = 102 °C

$n_D^{20}$ = 1,4855

Ausbeute = 82 % der Theorie

Beispiel A

Panonychus-Test

Lösungsmittel : 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, das die angegebene Menge Emulgator enthält, und verdünnt das Konzentrat mit Wasser auf die gewünschte Konzentration.

Mit der Wirkstoffzubereitung werden Pflaumenbäumchen (Prunus myrobolana), die ungefähr eine Höhe von 20 bis 30 cm haben, tropfnaß besprüht. Diese Pflaumenbäumchen sind stark mit allen Entwicklungsstadien der Obstbaumspinnmilbe (Panonychus ulmi) befallen.

Nach den angegebenen Zeiten wird die Wirksamkeit bestimmt, indem man die überlebenden Tiere auszählt. Der so erhaltene Wirkungsgrad wird in % angegeben. 100% bedeutet, daß alle und 0%, daß keine Spinnmilben abgetötet wurden.

Bei diesem Test zeigten z.B. die Verbindungen der Herstellungsbeispiele (1) und (13) bei einer beispielhaften Konzentration von 0,00016% nach 14 Tagen einen Abtötungsgrad von 100%.

Beispiel B

Panonychus-Test

Lösungsmittel : 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, das die angegebene Menge Emulgator enthält, und verdünnt das Konzentrat mit Wasser auf die gewünschte Konzentration.

20 bis 30 cm goße Pflaumenbäumchen (Prunus myrobolana), die mit etwa 100 Eiern der Obstbaumspinnmilbe (Panonychus ulmi) besetzt sind, werden durch Spritzen mit der Wirkstoffzubereitung der gewünschten Konzentration behandelt.

Nach den angegebenen Zeiten wird die Wirksamkeit bestimmt, indem man die überlebenden Tiere auszählt. Der so erhaltene Wirkungsgrad wird in % angegeben. 100% bedeutet, daß alle und 0%, daß keine Spinnmilben abgetötet wurden.

Bei diesem Test zeigten z.B. die Verbindungen der Herstellungsbeispiele (1) und (13) bei einer beispielhaften Konzentration von 0,00016% nach 12 Tagen einen Abtötungsgrad von 100%.

Beispiel C

Tetranychus-Test

Lösungsmittel : 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt mit Wasser auf die gewünschte Konzentration.

Bohnenpflanzen (Phaseolus vulgaris), die mit etwa 100 Eiern der Bohnenspinnmilbe (Tetranychus urticae) besetzt sind, werden durch Tauchen in die Wirkstoffzubereitung der gewünschten Konzentration behandelt.

Nach der gewünschten Zeit wird die Abtötung in % bestimmt. Dabei bedeutet 100%, daß alle, und 0%, daß keine Spinnmilben (Eier und Entwicklungsstadien) abgetötet wurden.

Bei diesem Test zeigten z.B. die Verbindungen der Herstellungsbeispiele (1) und (13) bei einer beispielhaften Konzentration von 0,0008% nach 12 Tagen eine Abtötung von 100%.

Beispiel D

Eisterilitäts- und Entwicklungshemmtest mit Tetranychus urticae (Gemeine Spinnmilbe)

Lösungsmittel : 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Die Blätter einer Bohnenpflanze (Phaseolus vulgaris) taucht man in die entsprechend konzentrierte Wirkstoffzubereitung. Nach Antrocknen der Wirkstoffzubereitung besetzt man die Blätter für ca. 16 Stunden mit Spinnmilben-Weibchen zur Eiablage (ca. 50 Eier/Wiederholung). Die Summe der sterilen und/oder abgetöteten Eier und der abgetöteten Larven, Nymphen und Ruhestadien einer Generation bezogen auf die Zahl der abgelegten Eier ergibt die Abtötung in %. Dabei bedeutet 100%, daß alle Tiere abgetötet wurden ; 0% bedeutet, daß keine Tiere abgetötet wurden.

Bei diesem Test zeigten z.B. die Verbindungen der Herstellungsbeispiele (1), (11), (13), (14), (16) und (17) bei einer beispielhaften Konzentration von 0,001% nach 12 Tagen eine Abtötung von 100%.

## Ansprüche

1. 1-Arylfurazanyl-3-(2-alkyl-4-halogenalkoxy-phenyl)-(thio)harnstoffe der allgemeinen Formel (I)

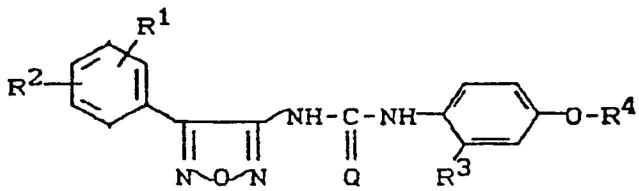

(I)

in welcher

| | |
|---|---|
| Q | für Sauerstoff oder Schwefel steht, |
| $R^1$ | für Wasserstoff, Halogen, Alkyl, Halogenalkyl, Alkoxy, Halogenalkoxy, Alkylthio oder Halogenalkylthio steht und |
| $R^2$ | für Wasserstoff, Halogen, Alkyl oder Alkoxy steht, oder |
| $R^1$ und $R^2$ | zusammen für gegebenenfalls durch Halogen substituiertes Alkylendioxy stehen, |
| $R^3$ | für Alkyl steht und |
| $R^4$ | für durch Fluor und gegebenenfalls zusätzlich durch Chlor und/oder Brom substituiertes Alkyl steht. |

2. Verbindungen gemäß Anspruch 1, in welchen

| | |
|---|---|
| Q | für Sauerstoff oder Schwefel steht, |
| $R^1$ | für Wasserstoff, Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_6$-Alkylthio oder für $C_1$-$C_4$-Halogenalkylthio steht und |
| $R^2$ | für Wasserstoff, Halogen, $C_1$-$C_4$-Alkyl oder $C_1$-$C_4$-Alkoxy steht, oder |
| $R^1$ und $R^2$ | zusammen für gegebenenfalls durch Fluor und/oder Chlor substituiertes $C_1$-$C_3$-Alkylendioxy stehen, |
| $R^3$ | für $C_1$-$C_4$-Alkyl steht und |
| $R^4$ | für durch Fluor und gegebenenfalls zusätzlich durch Chlor und/oder Brom substituiertes $C_1$-$C_6$-Alkyl steht. |

3. Verbindungen gemäß Anspruch 1, in welchen

| | |
|---|---|
| Q | für Sauerstoff oder Schwefel (vorzugsweise Sauerstoff) steht, |
| $R^1$ | für Wasserstoff, Fluor, Chlor, Brom, $C_1$-$C_4$Alkyl, für durch Fluor und/oder Chlor substituiertes Methyl, für $C_1$-$C_4$-Alkoxy, für durch Fluor und/oder Chlor substituiertes $C_1$-$C_3$-Alkoxy, für $C_1$-$C_4$-Alkylthio oder für durch Fluor und/oder Chlor substituiertes $C_1$-$C_4$Alkylthio steht und |
| $R^2$ | für Wasserstoff, Fluor, Chlor, Brom, Methyl oder Methoxy steht, oder |
| $R^1$ und $R^2$ | zusammen für gegebenenfalls durch Fluor und/ oder Chlor substituiertes $C_1$-$C_2$-Alkylendioxy stehen, |
| $R^3$ | für Methyl steht und |
| $R^4$ | für durch Fluor und gegebenenfalls zusätzlich durch Chlor substituiertes $C_1$-$C_4$-Alkyl steht. |

4. Verbindungen der Formel (I), in welcher

| | |
|---|---|
| $R^1$ | für Wasserstoff, Fluor, Chlor, Methyl, Trifluormethyl oder Methoxy steht, |
| $R^2$ | für Wasserstoff, Fluor oder Chlor steht, |
| $R^3$ | für Methyl steht, |
| $R^4$ | für $-CF_3$ oder $-CF_2CHFCl$ steht und |
| Q | für Sauerstoff oder Schwefel (vorzugsweise Sauerstoff) steht. |

5. Verfahren zur Herstellung der 1-Arylfurazanyl-3-(2-alkyl-4-halogenalkoxy-phenyl)-(thio)harnstoffe der allgemeinen Formel (I)

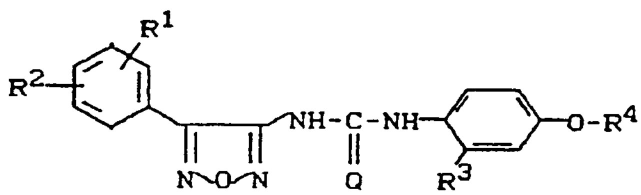

(I)

in welcher

| | |
|---|---|
| Q | für Sauerstoff oder Schwefel steht, |
| $R^1$ | für Wasserstoff, Halogen, Alkyl, Halogenalkyl, Alkoxy, Halogenalkoxy, Alkylthio oder Halogenalkylthio steht und |
| $R^2$ | für Wasserstoff, Halogen, Alkyl oder Alkoxy steht, oder |
| $R^1$ und $R^2$ | zusammen für gegebenenfalls durch Halogen substituiertes Alkylendioxy stehen, |
| $R^3$ | für Alkyl steht und |
| $R^4$ | für durch Fluor und gegebenenfalls zusätzlich durch Chlor und/oder Brom substituiertes Alkyl steht, |

dadurch gekennzeichnet, daß man

(a) 3-Amino-4-aryl-1,2,5-oxadiazole ("Arylfurazanylamine") der allgemeinen Formel (II),

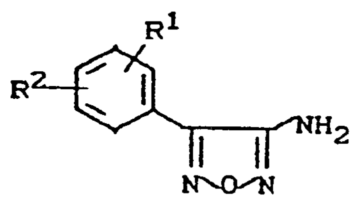

(II)

in welcher
$R^1$ und $R^2$ die oben angegebenen Bedeutungen haben,
mit 2-Alkyl-4-halogenalkoxy-phenyl-iso(thio)-cyanaten der allgemeinen Formel (III)

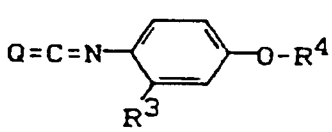

(III)

in welcher
Q, $R^3$ und $R^4$ die oben angegebenen Bedeutungen haben,
gegebenenfalls in Gegenwart von Katalysatoren und gegebenenfalls in Gegenwart von Verdünnungsmitteln umsetzt, oder

(b) 3-Iso(thio)cyanato-4-aryl-1,2,5-oxadiazole (Arylfurazanyliso(thio)cyanate") der allgemeinen Formel (IV)

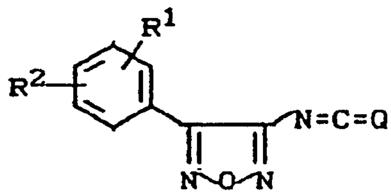

(IV)

in welcher
Q, $R^1$ und $R^2$ die oben angegebenen Bedeutungen haben,
mit 2-Alkyl-4-halogenalkoxy-anilinen der allgemeinen Formel (V)

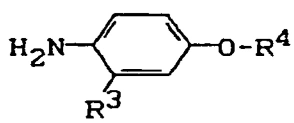

(V)

in welcher
$R^3$ und $R^4$ die oben angegebenen Bedeutungen haben,
gegebenenfalls in Gegenwart von Katalysatoren und gegebenenfalls in Gegenwart von Verdünnungsmitteln umsetzt.

6. Schädlingsbekämpfungsmittel, gekennzeichnet durch einen Gehalt an mindestens einer Verbindung der Formel (I), gemäß den Ansprüchen 1 oder 5.

7. Verwendung von Verbindungen der formel (I) gemäß den Ansprüchen 1 oder 5 zur Bekämpfung von Schädlingen.

8. Verfahren zur Bekämpfung von Schädlingen, dadurch gekennzeichnet, daß man Verbindungen der formel (I) gemäß den Ansprüchen 1 oder 5 auf Schädlinge und/oder ihren Lebensraum einwirken läßt.

9. Verfahren zur Herstellung von Schädlingsbekämpfungsmitteln, dadurch gekennzeichnet, daß man Verbindungen der formel (I) gemäß den Ansprüchen 1 oder 5 mit Streckmitteln und/oder oberflächenaktiven Mitteln vermischt.

**Claims**

1. 1-arylfurazanyl-3-(2-alkyl-4-halogenoalkoxy-phenyl)-(thio)ureas of the general formula (I)

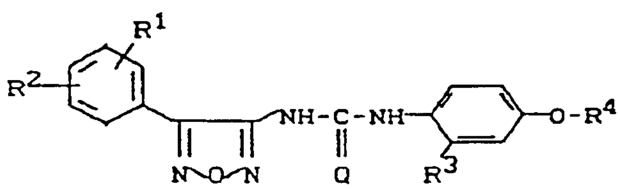

(I)

in which

| | |
|---|---|
| Q | stands for oxygen or sulphur, |
| $R^1$ | stands for hydrogen, halogen, alkyl, halogenoalkyl, alkoxy, halogenoalkoxy, alkylthio or halogenoalkylthio and |
| $R^2$ | stands for hydrogen, halogen, alkyl or alkoxy, or |
| $R^1$ and $R^2$ | together stand for alkylenedioxy which is optionally substituted by halogen, |
| $R^3$ | stands for alkyl and |
| $R^4$ | stands for alkyl which is substituted by fluorine and optionally in addition by chlorine and/or bromine. |

2. Compounds according to Claim 1, in which

| | |
|---|---|
| Q | stands for oxygen or sulphur, |

| | |
|---|---|
| $R^1$ | stands for hydrogen, halogen, $C_1$-$C_6$-alkyl, $C_1$-$C_4$-halogenoalkyl, $C_1$-$C_6$-alkoxy, $C_1$-$C_4$-halogenoalkoxy, $C_1$-$C_6$-alkylthio or for $C_1$-$C_4$-halogenoalkylthio and |
| $R^2$ | stands for hydrogen, halogen, $C_1$-$C_4$-alkyl or $C_1$-$C_4$-alkoxy, or |
| $R^1$ and $R^2$ | together stand for $C_1$-$C_3$-alkylenedioxy which is optionally substituted by fluorine and/or chlorine, |
| $R^3$ | stands for $C_1$-$C_4$-alkyl and |
| $R^4$ | stands for $C_1$-$C_6$-alkyl which is substituted by fluorine and optionally in addition by chlorine and or bromine. |

3. Compounds according to Claim 1, in which

| | |
|---|---|
| Q | stands for oxygen or sulphur (preferably oxygen), |
| $R^1$ | stands for hydrogen, fluorine, chlorine, bromine, $C_1$-$C_4$-alkyl, for methyl which is substituted by fluorine and/or chlorine, for $C_1$-$C_4$-alkoxy, for $C_1$-$C_3$-alkoxy which is substituted by fluorine and/or chlorine, for $C_1$-$C_4$-alkylthio or for $C_1$-$C_4$-alkylthio which is substituted by fluorine and/or chlorine and |
| $R^2$ | stands for hydrogen, fluorine, chlorine, bromine, methyl or methoxy, or |
| $R^1$ and $R^2$ | together stand for $C_1$-$C_2$-alkylenedioxy which is optionally substituted by fluorine and/or chlorine, |
| $R^3$ | stands for methyl and |
| $R^4$ | stands for $C_1$-$C_4$-alkyl which is substituted by fluorine and optionally in addition by chlorine. |

4. Compounds of the formula (I), in which

| | |
|---|---|
| $R^1$ | stands for hydrogen, fluorine, chlorine, methyl, trifluoromethyl or methoxy, |
| $R^2$ | stands for hydrogen, fluorine or chlorine, |
| $R^3$ | stands for methyl, |
| $R^4$ | stands for $-CF_3$, or $-CF_2CHFCl$ and |
| Q | stands for oxygen or sulphur (preferably oxygen). |

5. Process for the preparation of the 1-arylfurazanyl-3-(2-alkyl-4-halogenoalkoxy-phenyl)-(thio)ureas of the general formula (I)

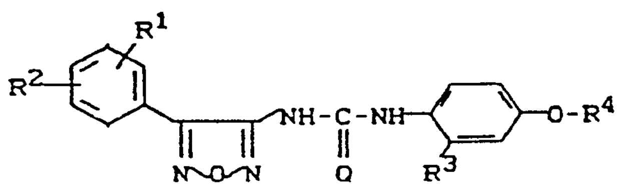

(I)

in which

| | |
|---|---|
| Q | stands for oxygen or sulphur, |
| $R^1$ | stands for hydrogen, halogen, alkyl, halogenoalkyl, alkoxy, halogenoalkoxy, alkylthio or halogenoalkylthio and |
| $R^2$ | stands for hydrogen, halogen, alkyl or alkoxy, or |
| $R^1$ and $R^2$ | together stand for alkylenedioxy which is optionally substituted by halogen, |
| $R^3$ | stands for alkyl and |
| $R^4$ | stands for alkyl which is substituted by fluorine and optionally in addition by chlorine and/or bromine, |

characterised in that

(a) 3-amino-4-aryl-1,2,5-oxadiazoles ("arylfurazanylamines") of the general formula (II),

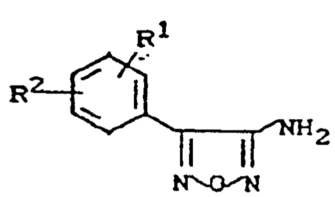

(II)

in which
$R^1$ and $R^2$ have the abovementioned meanings, are reacted with 2-alkyl-4-halogenoalkoxyphenyl-iso(thio)cyanates of the general formula (III)

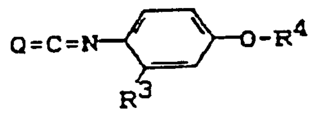

(III)

in which
Q, $R^3$ and $R^4$ have the abovementioned meanings, if appropriate in the presence of catalysts and if appropriate in the presence of diluents, or
(b) 3-iso(thio)cyanato-4-aryl-1,2,5-oxadiazoles ("arylfurazanyliso(thio)cyanates") of the general formula (IV)

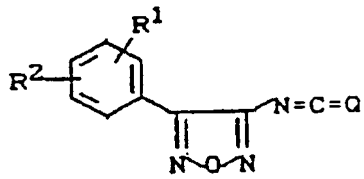

(IV)

in which
Q, $R^1$ and $R^2$ have the abovementioned meanings, are reacted with 2-alkyl-4-halogenoalkoxy-anilines of the general formula (V)

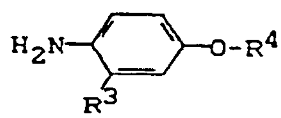

(V)

in which
$R^3$ and $R^4$ have the abovementioned meanings, if appropriate in the presence of catalysts and if appropriate in the presence of diluents.

6. Pesticides, characterised in that they contain at least one compound of the formula (I) according to Claims 1 or 5.

7. Use of compounds of the formula (I) according to Claims 1 or 5 for combating pests.

8. Method for combating pests, characterised in that compounds of the formula (I) according to Claims 1 or 5 are allowed to act on pests and/or their environment.

9. Process for the preparation of pesticides, characterised in that compounds of the formula (I) according to Claims 1 or 5 are mixed with extenders and/or surface-active agents.

## Revendications

1. 1-arylfurazanyl-3-(2-alkyl-4-halogénoalcoxy-phényl)-(thio)urées de formule générale (I)

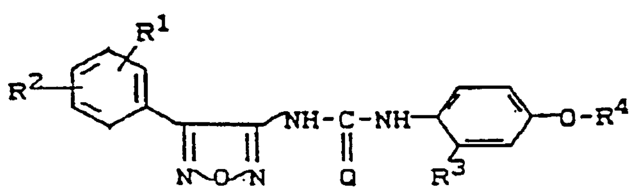

(I)

dans laquelle

Q représente l'oxygène ou le soufre,
$R^1$ représente l'hydrogène, un halogène, un groupe alkyle, halogénoalkyle, alcoxy, halogénoalcoxy,

alkylthio ou halogénoalkylthio, et

$R^2$ représente l'hydrogène, un halogène, un groupe alkyle ou un groupe alcoxy, ou

$R^1$ et $R^2$ représentent ensemble un groupe alkylènedioxy éventuellement substitué par un halogène,

$R^3$ représente un groupe alkyle et

$R^4$ représente un groupe alkyle substitué par le fluor et éventuellement de plus par le chlore et/ou le brome.

2. Composés selon la revendication 1, dans lesquels

Q représente l'oxygène ou le soufre,

$R^1$ représente l'hydrogène, un halogène, un groupe alkyle en $C_1$-$C_6$, halogénoalkyle en $C_1$-$C_4$, alcoxy en $C_1$-$C_6$, halogénoalcoxy en $C_1$-$C_4$, alkylthio en $C_1$-$C_6$ ou halogénoalkylthio en $C_1$-$C_4$, et

$R^2$ représente l'hydrogène, un halogène, un groupe alkyle en $C_1$-$C_4$ ou alcoxy en $C_1$-$C_4$, ou

$R^1$ et $R^2$ représentent ensemble un groupe alkylènedioxy en $C_1$-$C_3$ éventuellement substitué par le fluor et/ou le chlore,

$R^3$ représente un groupe alkyle en $C_1$-$C_4$ et

$R^4$ représente un groupe alkyle en $C_1$-$C_6$ substitué par le fluor et éventuellement de plus par le chlore et/ou le brome.

3. Composés selon la revendication 1, dans lesquels

Q représente l'oxygène ou le soufre (de préférence l'oxygène),

$R^1$ représente l'hydrogène, le fluor, le chlore, le brome, un groupe alkyle en $C_1$-$C_4$, un groupe méthyle substitué par le fluor et/ou le chlore, un groupe alcoxy en $C_1$-$C_4$, un groupe alcoxy en $C_1$-$C_3$ substitué par le fluor et/ou le chlore, un groupe alkylthio en $C_1$-$C_4$ ou un groupe alkylthio en $C_1$-$C_4$ substitué par le fluor et/ou le chlore, et

$R^2$ représente l'hydrogène, le fluor, le chlore, le brome, un groupe méthyle ou un groupe méthoxy, ou

$R^1$ et $R^2$ représentent ensemble un groupe alkylènedioxy en $C_1$-$C_4$ éventuellement substitué par le fluor et/ou le chlore,

$R^3$ représente un groupe méthyle et

$R^4$ représente un groupe alkyle en $C_1$-$C_4$ substitué par le fluor et éventuellement de plus par le chlore.

4. Composés de formule (I) dans laquelle

$R^1$ représente l'hydrogène, le fluor, le chlore, un groupe méthyle, trifluorométhyle ou méthoxy,

$R^2$ représente l'hydrogène, le fluor, ou le chlore,

$R^3$ représente un groupe méthyle,

$R^4$ représente $-CF_3$ ou $-CF_2CHFCl$ et

Q représente l'oxygène ou le soufre (de préférence l'oxygène).

5. Procédé pour la fabrication des 1-arylfurazanyl-3-(2-alkyl-4-halogénoalcoxy-phényl)-(thio)urées de formule générale (I)

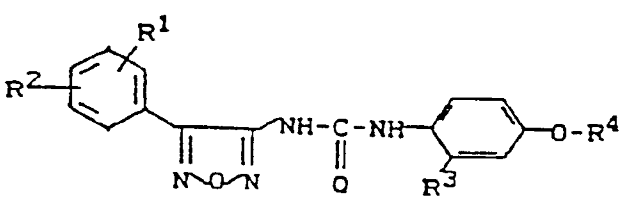

(I)

dans laquelle

Q représente l'oxygène ou le soufre,

$R^1$ représente l'hydrogène, un halogène, un groupe alkyle, halogénoalkyle, alcoxy, halogénoalcoxy, alkylthio ou halogénoalkylthio, et

| | |
|---|---|
| $R^2$ | représente l'hydrogène, un halogène, un groupe alkyle ou un groupe alcoxy, ou |
| $R^1$ et $R^2$ | représentent ensemble un groupe alkylènedioxy éventuellement substitué par un halogène, |
| $R^3$ | représente un groupe alkyle et |
| $R^4$ | représente un groupe alkyle substitué par le fluor et éventuellement de plus par le chlore et/ou le brome, |

caractérisé en ce que

(a) on fait réagir des 3-amino-4-aryl-1,2,5-oxadiazoles ("arylfurazanylamines") de formule générale (II),

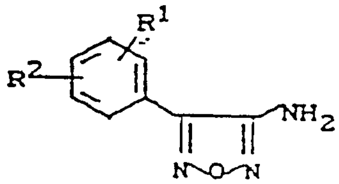

(II)

dans laquelle

$R^1$ et $R^2$ possèdent les significations décrites ci-dessus, avec des 2-alkyl-4-halogénoalcoxy-phényl-iso(thio)cyanates de formule générale (III)

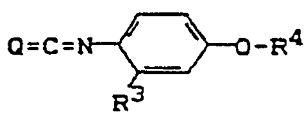

(III)

dans laquelle

Q, $R^3$ et $R^4$ ont les significations décrites ci-dessus, éventuellement en présence de catalyseurs et éventuellement en présence d'agents de dilution, ou

(b) on fait réagir des 3-iso(thio)cyanato-4-aryl-1,2,5-oxadiazoles ("arylfurazanyliso(thio)cyanates") de formule générale (IV)

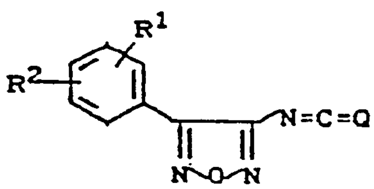

(IV)

dans laquelle

Q, $R^1$ et $R^2$ possèdent les significations décrites ci-dessus, avec des 2-alkyl-4-halogénoalcoxy-anilines de formule générale (V)

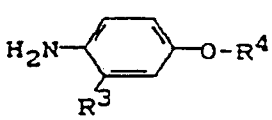

(V)

dans laquelle

$R^3$ et $R^4$ possèdent les significations décrites ci-dessus, éventuellement en présence de catalyseurs et éventuellement en présence d'agents de dilution.

6. Produits pour lutter contre les organismes nuisibles, caractérisés en ce qu'ils contiennent au moins un composé de formule (I) selon les revendications 1 ou 5.

7. Utilisation de composés de formule (I) selon les revendications 1 ou 5 pour la lutte contre les organismes nuisibles.

8. Procédé pour lutter contre les organismes nuisibles, caractérisés en ce qu'on fait agir des composés de formule (I) selon les revendications 1 ou 5 sur les organismes nuisibles et/ou sur leur habitat.

9. Procédé pour fabriquer des produits de lutte contre les organismes nuisibles, caractérisé en ce qu'on mélange des composés de formule (I) selon les revendications 1 ou 5 avec des agents d'allongement et/ou des agents tensio-actifs.